# EUROPEAN PATENT APPLICATION

(11) **EP 2 016 957 A1**
(43) Date of publication of application: **21.01.2009**
(21) Application number: 07014336.7
(22) Date of filing: 20.07.2007
(51) Int. Cl.: A61L 29/08, A61L 29/14, A61L 29/16

(54) **Drug-coated catheter and method for forming a drug-coated catherer**

(71) Applicant: Capsulution Nanoscience AG, 12489 Berlin (DE)
(72) Inventor: Kröhne, Lutz, 10439 Berlin (DE); Voigt, Andreas, Dr., 12621 Berlin (DE)
(74) Representative: Leidescher, Thomas

(57) **Abstract**

A catheter 10 comprises a surface 1 and a multilayer coating 3 on its surface 1. The multilayer coating 3 comprises alternate polymeric polyelectrolyte layers 7 and non-polymeric drug layers 6, wherein the non-polymeric drug layers 6 comprising a non-polymeric drug selected from the group containing antiseptic agents, anticoagulant drugs, anti-infectious agents, and mixtures thereof.

## Description

This description relates to embodiments pertaining to a catheter comprising a drug-containing multilayer coating and a method for coating a catheter.

### BACKGROUND OF THE INVENTION

Recently, bioactive coatings have been studied to evaluate their capability as drug carriers and as a coating for medical devices. In those systems, the drug is typically embedded in a polymer matrix. For delivery of therapeutics, approaches using drugs which are linked to polymers have also been studied. Many of the commonly available coatings, however, fail to provide a sustained release over a time period of few days or few weeks as desired for temporal application of catheters or the like.

### SUMMARY OF THE INVENTION

According to an embodiment, a method for coating a catheter is provided. The method comprises the steps of:
- providing a catheter comprising a surface; and
- forming a multilayer coating on the surface of the catheter by alternately depositing polymeric polyelectrolyte layers and non-polymeric drug layers, the non-polymeric drug layers comprising a non-polymeric drug selected from the group containing antiseptic agents, anticoagulant drugs, anti-infectious agents and mixtures thereof.

According to another embodiment, a catheter is provided which comprises
- a surface; and
- a multilayer coating on the surface of the catheter, the multilayer coating comprising alternate polymeric polyelectrolyte layers and non-polymeric drug layers, wherein the non-polymeric drug layers comprising a non-polymeric drug selected from the group containing antiseptic agents, anticoagulant drugs, anti-infectious agents and mixtures thereof.

In the context of this description, the term "non-polymeric drug layer" intends to describe a layer which is substantially formed by at least one non-polymeric drug. The term "substantially formed by a non-polymeric drug" as used herein intends to describe that the main component of the non-polymeric drug layer in terms of mass ratio is at least one non-polymeric drug. For example, the non-polymeric drug layer can be comprised of at least 50 wt. % of non-polymeric drug. A non-polymeric drug comprises at least two charges, which are typically of equal charge but which can also be amphoteric or of dual charge. Further, the non-polymeric drug or drugs are un-conjugated, i.e. they are not conjugated to other large molecules such as polymers but provided as such to form the non-polymeric drug layer.

The term "antiseptic agent", as used herein, includes compounds or entities which are fungicide or bactericide. An example is chlorhexidine. When referring to chlorhexidine, it is intended to include all pharmaceutically acceptable derivatives thereof. The term "anticoagulant drug" as used herein includes compounds or entities which prevent coagulation; that is, they stop blood from clotting. The term "anti-infectious agent" as used herein includes compounds that reduce or prevents infections. In the remainder of this description, the term drug is for antiseptic agents, anticoagulant drugs, anti-infectious agents, and mixtures thereof. In some embodiments, the drug has a molecular weight of less than about 2000 Da and particularly less than 1000 Da.

The term "polymeric polyelectrolyte layer" as used herein intends to describe a layer which is formed by at least one polymeric polyelectrolyte. Unlike the non-polymeric drug, polymeric polyelectrolytes comprise a plurality of covalently bounded monomers. Many of the monomers are charged. Typically, a polymeric polyelectrolyte carries at least 10 charges and typically more than 20 charges. The polymeric polyelectrolyte can be formed by polymerisation or cross-linking and have a molecular weight of at least 5000 Da, more particularly of about at least 10000 Da.

Unlike previous attempts, the multilayer coating as described herein is formed by alternately deposing layers comprised of a non-polymeric drug and layers comprised of polymeric polyelectrolytes. Such formed and composed multilayer coatings exhibit a sustained release characteristic of the drug for many days up to few weeks and more while maintaining sufficient stability on the catheter. The multilayer coating as described herein is therefore suitable for applications which require a sustained release for a given time.

### BRIEF DESCRIPTION OF THE DRAWINGS

A full and enabling disclosure of the present invention, including the best mode thereof, to one of ordinary skill in the art, is set forth more particularly in the remainder of the specification, including reference to the accompanying figures. Therein:

Figure 1 shows the chemical structure of chlorhexidine.

Figure 2 illustrates a multilayer coating comprising alternate polymeric polyelectrolyte layers and non-polymeric drug layers.

Figure 3 illustrates the experimental set-up for determining the release rate.

Figure 4 shows the formation of a multilayer coating monitored by QCM-D (Quartz Crystal Microbalance with Dissipation).

Figure 5 shows the release rate of chlorhexidine from a multilayer coating.

### DESCRIPTION OF THE PREFERRED EMBODIMENT

Reference will now be made in detail to various embodiments, one or more examples of which are illustrated in the figures. Each example is provided by way of explanation, and is not meant as a limitation of the invention. For example, features illustrated or described as part of one embodiment can be used on or in conjunction with other embodiments to yield yet a further embodiment. It is intended that the present invention includes such modifications and variations. The examples are described using specific language which should not be construed as limiting the scope of the appending claims. The drawings are not scaled and are for illustrative purposes only.

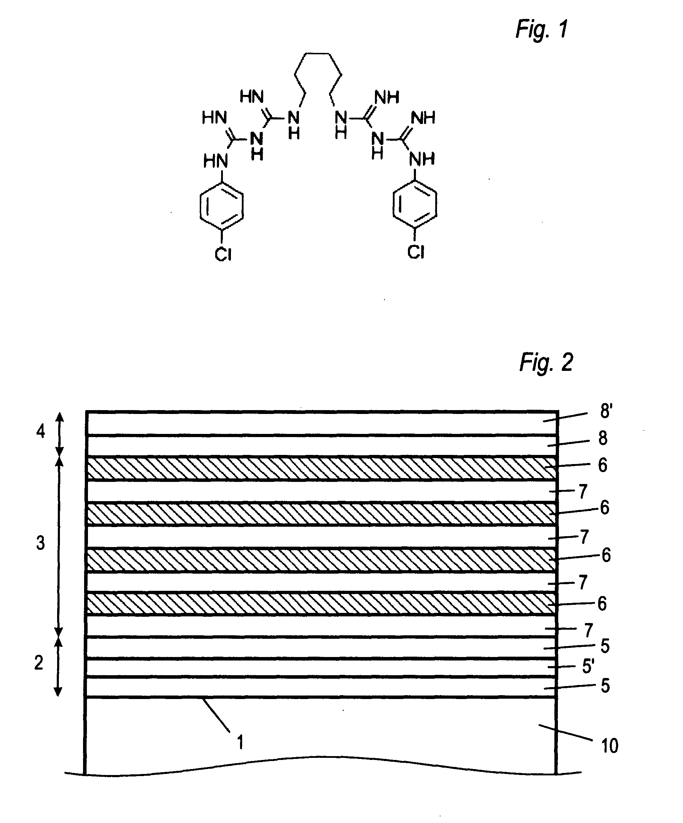

Figure 2 illustrates an embodiment of a catheter 10 with a multilayer coating 3. The catheter 10 comprises a surface 1. Surface 1 can refer to an outer, an inner or to both surfaces of the catheter 10. Figure 2 shows only a portion of a catheter and the multilayer coating 3.

The catheter 10 is typically comprised of a bio.logical inert material. Many polymers such as polyurethane are biological inert and can be used as catheter material. Furthermore, the catheter can be comprised of a biological inert metal.

The surface 1 of the catheter 10 is coated with the multilayer coating 3 which comprises a plurality of alternating layer 6, 7. In this embodiment, layers 7 refer to polymeric polyelectrolyte layers and layers 6 represent non-polymeric drug layers.

Typically, the alternating layers 6, 7 are of opposite charge. A layer stack comprising a polymeric polyelectrolyte layer 7 and a non-polymeric drug layer 6 of opposite charge are referred to as bi-layer. It is, however, also possible that a layer stack comprises a first polymeric polyelectrolyte layer having a given charge, a non-polymeric drug layer of opposite charge and a second polymeric polyelectrolyte layer of the same charge as the non-polymeric drug layer in this order. The non-polymeric drug layer and the second polymeric polyelectrolyte layer are considered here as one layer which comprises two sub-layers. The sub-layers are formed by material (non-polymeric drug and polymeric polyelectrolyte) having the same charge. In some embodiments, the multilayer coating 3 comprises at least two non-polymeric drug layers 6 and at least two polymeric polyelectrolyte layers 7. These embodiments therefore comprise at least two bi-layers as becomes apparent from the description below. In further embodiments, the multilayer coating 3 comprises at least three non-polymeric drug layers 6 and at least three polymeric polyelectrolyte layers 7. Hence, at least three bi-layers are comprised. In even further embodiments, the multilayer coating 3 comprises at least four non-polymeric drug layers 6 and at least four polymeric polyelectrolyte layers 7, which corresponds to four bi-layers. In other embodiments, number of the non-polymeric drug-layers 6 and the number of the polymeric polyelectrolyte layers 7 is not equal but can differ about 1, 2 or even more layers.

It has been surprisingly found that low-molecular weight molecules (drugs) are suitable to form multilayer coatings together with typically large (high-molecular weight) polymeric polyelectrolyte layers. Such multilayer coatings are stable for a sufficiently long time which makes them suitable for sustained drug delivery. It is assumed that the drug is slowly eluted from the multilayer coating. Since the drug itself forms layers which contribute to the multilayer coating arrangement, it is assumed that elution of the drug may result in a partial decomposition of the multilayer coating. The observed release rates of the drug are rather small so that a sustained release is observable over at least few days and up to few weeks. This is sufficient for the duration of a temporal implantation of a catheter.

Multilayers of alternately charged layers can be formed by a layer-by-layer (LbL) deposition method. Therefore, such multilayer arrangements are known as LbL-coating. The formation of such multilayer arrangements is based on self-assembling of the oppositely charged layers due to the electrostatic attraction.

The LbL-coating can optionally comprise a base layer 2 between the surface 1 of the catheter 10 and the multilayer coating 3. The base layer 2 is for improving the adhesion of the multilayer coating 3 on the surface 1. The base layer 2 can comprise at least two or at least three alternately charged polymeric polyelectrolyte layers 5, 5'. The base layer 2 can also comprise at least four polymeric polyelectrolyte layers. Experience has shown that at least four alternately charge polymeric electrolyte layers can form a sufficiently stable coating. It goes without saying that the higher the numbers of the layers the more stable the base layer 2 will be.

The LbL-coating can further optionally comprise a capping layer 4 on the multilayer coating 3. Typically, the capping layer 4 comprises at least two or at least three alternately charged polymeric polyelectrolyte layers 8, 8'. The capping layer 4 improves the stability of the multilayer coating 3. Again, the stability can be improved by increasing the number of the polymeric polyelectrolyte layers 8, 8'.

The polymeric polyelectrolyte layers of the base layer 2, the multilayer coating 3 and the capping layer 4 are comprised of polymeric polyelectrolytes having anionic or cationic character. Without being wished to be limited thereto, the polymeric polyelectrolytes can be selected from the group comprising alginate, carboxymethylcellulose, λ-Carrageenan, chondroitin 6-sulfate, dextran sulfate, lignosulfonic acid, poly-L-glutamic acide (PLGA), polyacrylic acide (PAA), polygalacturonic acid, poly(methacrylic acid), polystyrensulfonic acid (PSS), polyvinylsulfonic acid (PVS), sulfoethylcellulose, Xanthan gum, polyallylamine hydrochloride (PAH), polydiallyldimethylammoniumchloride (PDA), chitosan, protamin, biopolymers, collagen, albumin, biopolymer derivatives, gelatine, cellulose derivatives, aminodextranes, polyarginine, polyethylenimine, polylysine, protamine or other cationic charged polymers, any salt of the above polymers, and mixtures thereof.

The base layer 2, the multilayer coating 3 and the capping layer 4 can comprise the same or different polymeric polyelectrolyte layers. In principle, any combination is possible. Further, even within each of the base layer 2, multilayer coating 3 and capping layer 4 different combinations of polymeric polyelectrolyte layers can be used. The multilayer coating forms together with the optional base land capping layer the LbL-coating.

The non-polymeric drug layers are substantially comprised of an antiseptic agent, an anticoagulant drug and/or an anti-infectious agent. In the specific examples given below, chlorhexidine has been used as a drug for forming the non-polymeric drug layers 6. Examples for anti-infectious agents such as antibiotics include, without being limited thereto, colloidal silver, carboxypenicillin, aminopenicillin, ampicillin, cephalosporin, cefsulodin, ceftazidim, ceflaclor, cefalexin, cephalosporin C, cilastatin, aztreonam, streptomycin, neomycin B, kanamycin A, trimethoprim, tetroxoprim, glycopeptides, vancomycin, teicoplanin, p-aminosalicylic acid, diaphenylsulfon, pyrimethamin, suramin, melasoprol, stibogluconat-sodium, pentamidin, foscarnet, aciclovir.

Subsequently, the formation of the multilayer coating will be described.

Prior to coating the surface 1 of the catheter 10, the surface 1 can be activated. For example, the catheter 10 can be placed in an activating solution for 10 minutes. For activating the inner surface of the catheter 10, the activating solution can be moved through the catheter 10 by syringe rinsing of the catheter inside at about every minute. Examples of chemical activation solutions are listed below in Table 1. Without being limited to chemical activation, other types of activation can be used which comprise plasma etching or radiation activation, e.g. ultra violet activation.

**Table 1**

| Composition | Characteristic |
|---|---|
| 35ml H₂O, 7ml H₂O₂ and 7ml HCl | Soft acid |
| 7ml H₂O₂ and 7ml HCl | strong acid |
| 35ml H₂O, 7ml H₂O₂ and 7ml NH₃ (25%) | soft base |
| 7ml H₂O₂ and 7ml NH₃ (25%) | strong base |

In a subsequent step, the optional base layer 2 is formed by alternately depositing polymeric polyelectrolyte layers 5, 5' of opposite charge. The polyelectrolyte layers 5, 5' can be deposited by dipping, spraying or rinsing.

After forming the base layer 2, the multilayer coating 3 is formed by alternately deposition of polymeric polyelectrolyte layers 7 and non-polymeric drug layers 6. For example, if the drug is a cation, the polyelectrolytes forming the polymeric polyelectrolyte layers are anions. It is also possible to deposit an anionic polymeric polyelectrolyte layer followed by deposition of a cationic drug and a cationic polymeric polyelectrolyte layer. Irrespective of the ionic nature of the drug and the polyelectrolyte, the above described deposition results in the formation of a bi-layer which comprises a first polymeric polyelectrolyte layer and a non-polymeric drug layer of opposite charge (polymer-drug bi-layer), or which comprises a first polymeric polyelectrolyte layer of a first charge, a non-polymeric drug layer of a second charge and a second polymeric polyelectrolyte layer of the second charge (polymer-drug-polymer bi-layer). The bi-layer formation is repeated to obtain the multilayer coating 3. Typically, the multilayer coating 3 comprises at least 5 bi-layers and particularly at least 10 bi-layers. In some embodiments, the multilayer coating 3 comprises at least 20 bi-layers which can be of different type, i.e. different combination of drug and polymeric polyelectrolyte or polyelectrolytes (different bi-layer combinations). As becomes apparent from the examples below, the multilayer coating 4 can also comprise polymer-polymer bi-layers to improve stability of the multilayer coating. Again, the layers 6, 7 can be deposited by dipping, spraying or rinsing.

Subsequently, the optional capping layer 4 is formed by depositing alternately charge polymeric polyelectrolyte layers 8, 8'. Again, the layers 8, 8' can be deposited by dipping, spraying or rinsing and different bi-layers can be used.

In specific examples, catheters comprised of polyurethane were used to form a multilayer coating 3 and to determine the release rate of the drug. In a first step the catheter surface was careful activated with respect to the subsequent polymer composition and multilayer coating. Catheters were placed in cylindrical tubes filled with cleaning/activating solutions (25 ml H₂O₂ and 25 ml NH₃ (25%)) for 10 minutes. During this time frame syringe rinsing of the catheter inside was performed at about every minute.

For the layer-by-layer coating (LbL) the polyelectrolytes were dissolved at 1 mg/mL and the drug (chlorhexidine, CHX) at 2 mg/mL in water at pH of choice. The LbL/drug films were prepared by sequential dipping cycles. Rinsing steps with water were carried out between subsequent coating cycles. In the time frame of each coating/ rinsing the fluid inside the catheters were changed by syringe action at about every minute. After finishing the coating/ rinsing cycles the catheters were air-dried in a flue. It is also possible to dry the deposited layers between deposition steps to increase the load of the deposited material. This is particularly of advantage for the non-polymeric drug layers.

The drug release rate of LbL coated catheters was investigated at 37°C. As the release medium, water with 2g/L sodium gluconate was used. Sodium gluconate was used for increasing the solubility of CHX (more than 20 g/L). The pH of the release medium was adjusted to pH 7 with acetic acid and/ or NaOH. To analyze the drug release rate the release medium (8.5 ml) was pumped with a low flow rate (about 4.9 ml/hour) through the catheter in a closed system loop. At given time intervals samples were taken to analyze the drug content with HPLC. The removed sample volume was replaced by fresh release medium.

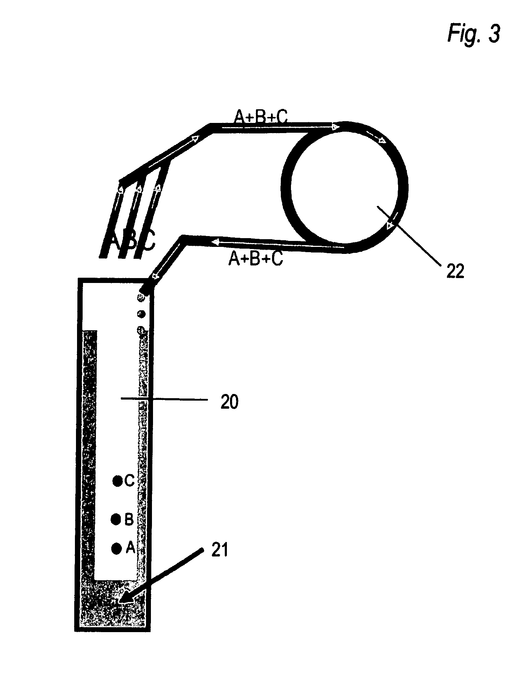

The experimental set-up for measuring the release rate is illustrated in Figure 3. A catheter 20, which comprised in this specific example three internal lumens, was brought into a release solution 21. The three lumens are denoted by A, B and C, respectively. The release solution 21 was pumped through the lumens by a pump 22.

### Examples

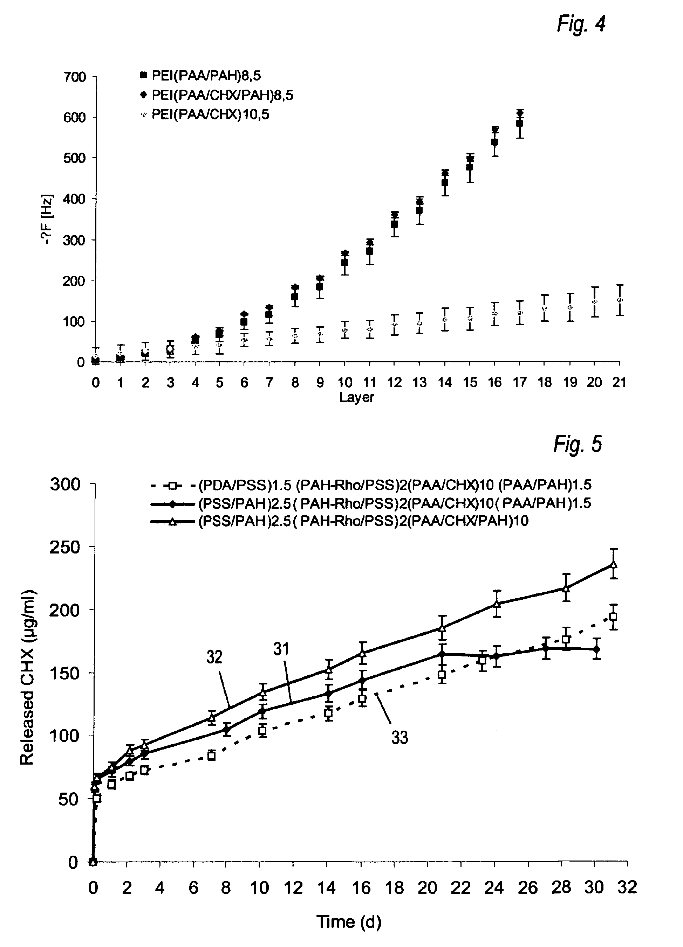

The catheters were activated at room temperature with a mixture of 25 ml H₂O₂ and 25 ml NH₃ (25%) for 10 minutes. After rinsing with water the LbL multilayer coating were constructed with PSS (Polystyrensulfonic acid) and PAH (polyallylamine hydrochloride) onto activated catheters surface. Between each deposition step the films were three times rinsed with water. As indicated in table 1, base layer 2 comprised two different types of bi-layers. After depositing nine polyelectrolyte layers, CHX was used as cationic component in combination with PAA as polyanionic component (Example 1, curve 31 in Figure 5). The capping layer 3 was formed by a PAA/PAH/PAA arrangement.

In the second example PAH was used as second cationic component after the CHX-layer (without washing between CHX and PAH, Example 2, curve 32 in Figure 5). This means that polymer-drug-polymer bi-layers were formed. No capping layer was formed in this example.

Example 3 uses again a polymer-drug bi-layer for forming the multilayer coating. The overall coatings are listed in table 2. The CHX release from these two coatings was measured (Figure 5).

**Table 2**

| **Example** | **Lbl Coating** | | |
|---|---|---|---|
| | **Base Layer** | **Multilayer coating** | **Capping layer** |
| 1 (curve 31) | (PSS/PAH)₂.₅ (PAH-Rho/PSS)₂ | (PAA/CHX)₁₀ | (PAA/PAH)_{1.5} |
| 2 (curve 31) | (PSS/PAH)_{2.5} (PAH-Rho/PSS)₂ | (PAA/CHX/PAH)₁₀ | none |
| | | | |
| 3 (curve 33) | (PDA/PSS)_{1.5} (PAH-Rho/PSS)₂ | (PAA/CHX)₁₀ | (PAA/PAH)_{1.5} |

Table 3 lists other possible coatings. As becomes apparent from table 3, example 8, the multilayer coating comprise polymer-polymer bi-layers arranged between polymer-drug bi-layers to improve stability of the multilayer coating.

**Table 3**

| **Example** | **Lbl Coating** | | |
|---|---|---|---|
| | **Base Layer** | **Multilayer coating** | **Capping layer** |
| 4 | (PDA/PSS)_{1.5} (PAH-Rho/PSS)₂ | (PAA/CHX/PAH)₁₀ | none |
| 5 | (PSS/PAH)_{2.5} (PAH-Rho/PSS)₂ | (PAA/CHX)₁₀ | (PAA/PAH)_{1.5} |
| 6 | (PSS/PAH)_{2.5} (PAH-Rho/PSS)₂ | (PAA/CHX)₂₀ | (PAA/PAH)_{1.5} |
| 7 | (PSS/PAH)_{2.5} (PAH-Rho/PSS)₂ | (PAA/CHX)₃₀ | (PAA/PAH)₁ |
| 8 | (PSS/PAH)_{2.5} (PAH-Rho/PSS)₂ | (PAA/CHX)₅ | (PAA/PAH)_{1.5} |
| | | (PAA/PAH)₂ | |
| | | (PAA/CHX)₅ | |
| | | (PAA/PAH)₂ | |
| | | (PAA/CHX)₅ | |
| | | (PAA/PAH)₂ | |
| | | (PAA/CHX)₅ | |

In the above examples, PAH-Rho refers to Rhodamine labelled PAH used for observing the assembly of the LbL coating. The assembling of the LbL coating can also be observed by QCM-D as shown in Figure 4 for different multilayer coating arrangements. A PAA/PAH multilayer, which does not comprise non-polymeric drug layers have been used as reference. As can be seen from Figure 4, the weight of a PAA/CHX multilayer increases slower in comparison with the PAA/PAH reference or a PAA/CHX/PAH multilayer coating due to the low molecular weight of the CHX in comparison with the high molecular weight of the polyelectrolytes.

All examples show a sustained release rate of CHX for at least 21 days and for even more than 4 weeks.

The written description above uses specific embodiments to disclose the invention, including the best mode, and also to enable any person skilled in the art to make and use the invention. While the invention has been described in terms of various specific embodiments, those skilled in the art will recognise that the invention can be practiced with modification within the spirit and scope of the claims. Especially, mutually non-exclusive features of the embodiments described above may be combined with each other. The patentable scope is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

### Reference list

US 2004/0241202 A1
US 5 788 959
WO 93/08766
R. Collin, "Decreasing Catheter Colonization Through the Use of an antiseptic-Impregnated catheter", Chest 1999, 115, 1632-1640
S. Schmitt et al., "Impact of Impact of Chlorhexidine-Silver Sulfadiazine-Impregnated Central Venous Catheters on In Vitro Quantitation of Catheter-Associated Bacteria", Journal of Clinical Microbiology 1996, 34(3), 508-511
Seymour S. Block, "Disinfection, Sterilization, and Preservation", Fifth Edition,
D.L. Veenstra et al., "Cost-Effiectiveness of Antiseptic-Impregnated Central Venous Catheters for the Prevention of Catheter-Related Bloodstream Infection", Journal of the American Medical Association, 1999, 282(6), 544-560

## Claims

1. Method for coating a catheter, comprising the steps of:
- providing a catheter comprising a surface; and
- forming a multilayer coating on the surface of the catheter by alternately depositing polymeric polyelectrolyte layers and non-polymeric drug layers, the non-polymeric drug layers comprising a non-polymeric drug selected from the group containing antiseptic agents, anticoagulant drugs, anti-infectious agents, and mixtures thereof.

2. Method of claim 1, wherein the non-polymeric drug has a molecular weight of less than 2000 Da.

3. Method of-claim 1 or 2, wherein the non-polymeric drug is selected from the group containing chlorhexidine, low-molecular heparin, colloidal silver, carboxy penicillin, aminopenicillin, ampicillin, cephalosporin, cefsulodin, ceftazidim, ceflaclor, cefalexin, cephalosporin C, cilastatin, aztreonam, streptomycin, neomycin B, kanamycin A, trimethoprim, tetroxoprim, glycopeptides, vancomycin, teicoplanin, p-aminosalicylic acid, diaphenylsulfon, pyrimethamin, suramin, melasoprol, stibogluconat-sodium, pentamidin, foscarnet, aciclovir, and mixtures thereof.

4. Method of any of the preceding claims, comprising the further step of:
- activating the surface of the catheter prior to forming the multilayer coating.

5. Method of any of the preceding claims, comprising the further step of:
- forming a base layer on the surface of the catheter prior to forming the multilayer coating, the base layer comprising at least two alternately charged polymeric polyelectrolyte layers.

6. Method of any of the preceding claims, comprising the further step of:
- forming a capping layer on the multilayer coating, the capping layer comprising at least two alternately charged polymeric polyelectrolyte layers.

7. Catheter, comprising:
- a surface; and
- a multilayer coating on the surface of the catheter, the multilayer coating comprising alternate polymeric polyelectrolyte layers and non-polymeric drug layers, wherein the non-polymeric drug layers comprising a non-polymeric drug selected from the group containing antiseptic agents, anticoagulant drugs, anti-infectious agents, and mixtures thereof.

8. Catheter of claim 7, wherein the non-polymeric drug has a molecular weight of less than 2000 Da.

9. Catheter of claim 7 or 8, wherein the non-polymeric drug is selected from the group containing chlorhexidine, low-molecular heparin, colloidal silver, carboxypenicillin, aminopenicillin, ampicillin, cephalosporin, cefsulodin, ceftazidim, ceflaclor, cefalexin, cephalosporin C, cilastatin, aztreonam, streptomycin, neomycin B, kanamycin A, trimethoprim, tetroxoprim, glycopeptides, vancomycin, teicoplanin, p-aminosalicylic acid, diaphenylsulfon, pyrimethamin, suramin, melasoprol, stibogluconat-sodium, pentamidin, foscarnet, aciclovir, and mixtures thereof.

10. Catheter of any of the claims 7 to 9, further comprising:
- a base layer arranged between the surface of the catheter and the multilayer coating, the base layer comprising at least two alternately charged polymeric polyelectrolyte layers.

11. Catheter of any of the claims 7 to 10, further comprising:
- a capping layer on the multilayer coating, the capping layer comprising at least two alternately charged polymeric polyelectrolyte layers.

12. Use of the catheter of any of the claims 7 to 11 or of the catheter coated by any of the claims 1 to 6 for localised therapy or temporal implantation.
